# FASCICULE DE BREVET EUROPEEN

(11) **EP 1 521 574 B1**
(45) Date de publication et mention de la délivrance du brevet: **07.03.2007**
(21) Numéro de dépôt: 03760779.3
(22) Date de dépôt: 24.06.2003
(51) Int. Cl.: A61K 9/16, A61K 9/20

(54) **COMPOSITION PHARMACEUTIQUE SOLIDE CONTENANT UN PRINCIPE ACTIF LIPOPHILE, SON PROCEDE DE PREPARATION**
EINEN LIPOPHILEN WIRKSTOFF ENTHALTENDE FESTE PHARMAZEUTISCHE ZUSAMMENSETZUNG SOWIE DEREN HERSTELLUNG
SOLID PHARMACEUTICAL COMPOSITION CONTAINING A LIPOPHILIC ACTIVE PRINCIPLE AND PREPARATION METHOD THEREOF

(30) Priorité: 25.06.2002 FR 0207831
(43) Date de publication de la demande: 13.04.2005
(73) Titulaire: CLL PHARMA, 06200 Nice (FR)
(72) Inventeur: ABOU CHACRA-VERNET, Marie-Line, Les Bergeronnettes, 06200 Nice (FR); ZAKARIAN, Noël, 13009 Marseille (FR); TOSELLI, Dominique, 06000 Nice (FR); GIMET, René, 06560 Valbonne (FR); LARUELLE, Claude, 06270 Villeneuve-Loubet (FR)
(74) Mandataire: Goulard, Sophie
(86) Numéro de dépôt international: PCT/FR2003/001933
(87) Numéro de publication internationale: WO 2004/000279

(56) Documents cités:
- EP-A- 0 410 422
- WO-A-99/33467
- FR-A- 2 758 459

## Description

La présente Invention se rapporte à une composition pharmaceutique solide, destinée à une administration par voie orale, comprenant au sein d'une seule et même phase, un principe actif lipophile, un agent tensioactif, un polymère cationique insoluble dans l'eau à pH neutre ou alcalin et un acide minéral ou organique, ainsi qu'à son procédé de préparation.

Une telle composition permet en particulier de conférer à ce type de principe actif, lorsqu'il est absorbé par la voie orale, une biodisponibilité significativement améliorée du fait d'une dissolution favorisée en milieu aqueux.

Il existe un grand nombre de principes actifs lipophiles présentant une faible hydrosolubilité et dont la formulation, pour une administration par la voie orale, pose un certain nombre de problèmes majeurs, notamment en termes de biodisponibilité.

Parmi ces principes actifs lipophiles figurent notamment la famille des hypolipidémiants à laquelle appartiennent en particulier les fibrates tels que le fénofibrate (DCI).

Le fénofibrate a été mis sur le marché pour la première fois en France en 1975, sous la forme de gélules dosées à 100 mg de principe actif (Lipanthyl® 100) et avec une posologie de 3 ou 4 gélules par jour à prendre en cours des différents repas, correspondant donc à une administration quotidienne de 300 à 400 mg de principe actif. Cette spécialité pharmaceutique est toujours commercialisée à ce jour en France mais sous le nom Sécalip® 100.

Depuis cette première mise sur le marché, les formulateurs n'ont pas cessé de chercher à améliorer la formulation des principes actifs lipophiles et en particulier celle du fénofibrate.

En 1986, un deuxième dosage, destiné à offrir aux patients ne nécessitant que 300 mg de fénofibrate par jour une meilleure observance thérapeutique, a été commercialisé sous la forme de gélules renfermant 300 mg de principe actif (Lipanthyl® 300), la posologie étant alors d'une seule gélule par jour. Une telle posologie est rendue possible par la longue durée d'action du fénofibrate dont la demi-vie d'élimination est, en effet, d'environ 20 heures. Cette spécialité pharmaceutique est toujours disponible en France, mais sous le nom Sécalip® 300.

Toutefois, cette forme galénique conduit à une faible biodisponibilité du principe actif (Desager *et al.,* J. Clin. Pharmacol., 1978, **26**, 570-574 ; Weil *et al.,* Drug Metabolism, 1980, 18, 115-120) ; Strolin Benedetti *et al.,* (Acta Pharmacol. Toxicol., 1986, **59**, suppl. 5, 1967). En effet, du fait de sa faible hydrosolubilité, le fénofibrate, et de façon plus générale, les principes actifs lipophiles, très peu solubles, voire insolubles dans l'eau, sont mal absorbés au niveau du tube digestif et présentent par conséquent une biodisponibilité incomplète.

Pour améliorer la dissolution du fénofibrate et sa biodisponibilité et réduire ainsi la dose devant être administrée, la demande de brevet FR-A-2 627 696 propose notamment une composition pharmaceutique dans laquelle le fénofibrate est utilisé sous la forme d'une poudre co-micronisée avec un tensioactif solide, par exemple du laurylsulfate de sodium. La poudre de fénofibrate co-micronisée ainsi obtenue est mélangée avec des excipients classiques tels que le lactose, l'amidon, la polyvinylpyrrolidone (PVP) et le stéarate de magnésium puis mise en gélule.

Ce brevet enseigne en particulier que la co-micronisation du fénofibrate avec un tensioactif solide permet d'améliorer la biodisponibilité du fénofibrate de façon significativement plus importante que l'amélioration que l'on obtiendrait soit par addition d'un agent tensioactif, soit en micronisant uniquement le fénofibrate, soit encore en mélangeant intimement le fénofibrate et le tensioactif micronisés séparément. Ce procédé conduit à une nouvelle forme galénique où le produit actif, co-micronisé avec un tensioactif solide, présente une dissolution du fénofibrate améliorée, donc une biodisponibilité augmentée, ce qui permet, à efficacité égale, une diminution de la dose quotidienne de médicament : respectivement 67 mg (Lipanthyl® 67 M ou Tricor®) et 200 mg (Lipanthyl® 200 M ou Tricor®) au lieu de 100 mg et 300 mg. Ainsi le patient a pu disposer d'une forme galénique ne nécessitant qu'une seule prise par jour qui produit un effet identique à celui obtenu à l'aide de prises multiples. Cependant, le procédé de préparation selon ce brevet n'est pas totalement satisfaisant dans la mesure où il ne conduit pas à une dissolution et à une biodisponibilité complète du principe actif.

La demande de brevet EP 0 793 958 décrit un procédé de préparation d'une composition pharmaceutique mettant en oeuvre du fénofibrate, un agent tensioactif et de la PVP. Selon ce document, les particules de fénofibrate sont mélangées avec une solution de PVP. Le mélange obtenu est ensuite granulé avec une solution de un ou plusieurs tensioactifs. Le granulé ainsi produit est alors séché.

La demande de brevet FR-A-2 783 421 montre que l'on peut aussi améliorer la biodisponibilité du fénofibrate à l'aide d'un procédé de préparation consistant à procéder à la micronisation du fénofibrate, puis à sa granulation en présence d'un milieu liquide comprenant un agent tensioactif, de l'eau et un alcool hydromiscible et enfin à sécher le granulé ainsi obtenu.

Selon la demande de brevet EP 1 048 295, la dissolution d'une composition de ce principe actif peut également être améliorée lorsque l'on réalise la co-micronisation du fénofibrate avec un excipient solide autre qu'un agent tensioactif, tel que le lactose, la cellulose microcristalline ou l'amidon.

De même, la demande internationale WO 02/11699 décrit une formulation galénique comprenant du fénofibrate micronisé, intimement mélangé à des excipients inertes tels que le lactose et la cellulose microcristalline dont la taille des particules (inférieure à 50 µm) est sélectionnée de sorte à éviter l'agrégation des particules de fénofibrate et à optimiser leur dispersion. Ainsi, la dissolution du fénofibrate est améliorée et la biodisponibilité de la composition ainsi préparée est supérieure à celle de la spécialité pharmaceutique Tricor® dosée à 200 mg et 67 mg. Toutefois, la dissolution du fénofibrate à 30 minutes n'est pas proche de 100 %.

Le brevet US 6,180,138 décrit un procédé de préparation d'une formule solide d'agent de régulation des lipides incluant le fénofibrate dont les caractéristiques de dissolution et d'absorption sont améliorées. Le procédé décrit dans ce brevet consiste en un mélange de fénofibrate co-micronisé avec éventuellement un ou plusieurs excipients tels que le lactose que l'on met en suspension dans une solution de tensioactif. Un électrolyte du type chlorure de sodium peut être ajouté à la solution de tensioactif de manière à éviter l'agglomération des particules de fénofibrate par force électrostatique. La suspension est ensuite séchée par atomisation ou en lit d'air fluidisé (LAF) et optionnellement granulée et mise sous forme de gélule ou de comprimé.

Le brevet US 6,368,622 se rapporte à un autre procédé de préparation d'une formule solide de fénofibrate consistant à fondre et à mélanger du fénofibrate et un tensioactif avec éventuellement un autre excipient. Le mélange congelé, broyé et réduit en poudre ou petits granulés est ensuite mis en gélules. Ce procédé permet d'obtenir de très petites particules de fénofibrate de manière à augmenter la surface spécifique et donc la dissolution. Les résultats de dissolution montrent une équivalence entre les compositions pharmaceutiques obtenues selon le procédé dans ce brevet et la spécialité Lipanthyl® 67 M.

Le brevet US 6,368,620 concerne un procédé de préparation d'une formulation galénique comprenant un agent de régulation du métabolisme lipidique incluant le fénofibrate, dissout dans du CO₂ supercritique et dont la solution est pulvérisée à travers une buse pour former de petites particules (nanoparticules ou nanocristaux) facilement dissoutes. L'ajout d'un agent de revêtement de surface, avant ou après l'étape de formation des nanoparticules, permet d'éviter l'agglomération des particules entre elles. Une suspension est ensuite formée pour collecter les particules et les sécher. La dissolution des formulations galéniques des nanoparticules ou nanocristaux ainsi générées est comparée à celle du Lipanthyl®.

Une autre méthode pour améliorer la dissolution du fénofibrate est décrite dans la demande de brevet EP 0 904 781. Il s'agit d'un procédé de préparation de granulés de fénofibrate, à partir d'une dispersion solide, lequel procédé comprend le mélange d'un agent désintégrant solide avec du fénofibrate fondu, le refroidissement et la solidification du mélange et la production de granulés par tamisage. Les désintégrants utilisés sont des polymères tels que l'amidon, la croscarmellose sodique, le glycolate d'amidon sodique et la PVP réticulée (crospovidone). Le granulé est ensuite introduit dans les compositions pharmaceutiques. Cependant, un désintégrant comme la crospovidone ne se dissout pas uniformément dans le fénofibrate fondu et présente des problèmes de compatibilité de phases. Ces phénomènes ont été mis en évidence par Sheu, M T *et al.,* Int. J. Pharm 1994, **103** (2), 137-146 en utilisant des mesures calorimétriques différentielles. Ces derniers ont aussi découvert que le fénofibrate est incompatible avec la PVP. Ainsi, la préparation de mélanges de matière pharmaceutique par fusion suivie d'une solidification conduit à une répartition et à une composition non uniforme dans le granulé final pouvant entraîner des effets indésirables sur la biodisponibilité du principe actif. De plus, le procédé de "co-fusion" nécessite l'utilisation d'équipements spécifiques.

La demande de brevet FR-A-2 722 984 décrit une dispersion solide de principes actifs assurant une meilleure solubilité dans les milieux aqueux et une meilleure résorption dans le tube digestif. La dispersion solide est préparée en dissolvant le principe actif dans un solvant organique volatil contenant un polymère très hydrophile tel qu'un amide cyclique, et éventuellement un tensioactif puis en évaporant à sec la solution organique. Le co-précipité du principe actif et du polymère ainsi formé est ensuite broyé, tamisé et dilué avec un excipient ou un véhicule pharmaceutiquement acceptable, ou bien utilisé tel quel. Il s'agit d'une composition comprenant un tensioactif, un principe actif dispersé dans un excipient hydrophile du type pyrrolidone.

La demande internationale WO 00/72829 décrit une composition pharmaceutique comprenant un agent de régulation lipidique incluant le fénofibrate et un excipient. Dans cette composition pharmaceutique, le fénofibrate et ledit excipient, par exemple l'acide succinique ou le polyéthylèneglycol (PEG) forment un mélange eutectique. Toutefois, ces dispersions solides ne présentent pas un profil de dissolution proche de 100 % et demandent des équipements spécifiques pour leur préparation.

La demande de brevet FR-A-2 758 459 décrit une composition pharmaceutique solide de fénofibrate ayant une biodisponibilité élevée ainsi que son procédé de préparation. Ce document décrit en particulier une composition de fénofibrate à libération immédiate comprenant un support inerte hydrosoluble enrobé d'une couche de fénofibrate micronisé dont la taille de particules est de moins de 20 µm, d'un polymère hydrophile tel que la PVP et éventuellement d'un agent tensioactif. Le microgranule comprend ensuite une phase externe ou un enrobage externe supplémentaire tel que par exemple de la PVP réticulée. La composition ainsi préparée montre une augmentation significative de la dissolution du fénofibrate. Même si l'augmentation du taux de dissolution *in vitro* ne signifie pas directement une augmentation de biodisponibilité *in vivo,* l'enseignement de ce document réside dans l'utilisation d'un polymère hydrophile et d'un support inerte hydrosoluble permettant d'organiser les particules de fénofibrate autour d'un support de manière à offrir au milieu de dissolution une plus grande surface spécifique. Dans ce cas de figure les profils de dissolution sont proches de 100 % puisque l'on obtient la dissolution suivante pour le comprimé à libération modifiée, dosé à 160 mg : 83 % de fénofibrate dissous en 15 minutes et 96 % en 30 minutes contre respectivement 16,5 % et 55 % pour le Lipanthyl® 200 M. Cette nouvelle composition pharmaceutique est actuellement commercialisée en France sous le nom Lipanthyl® 160 mg. Cependant, le procédé de mise en oeuvre est complexe, coûteux et demande des équipements spécifiques.

Il existe donc un besoin pour améliorer la dissolution et la biodisponibilité des principes lipophiles, très peu solubles, voire insolubles dans l'eau, et en particulier, celle des hypolipidémiants tels que le fénofibrate, sous forme solide, afin d'atteindre dans des temps très courts un niveau proche de 100 %, en tout cas supérieur à 80 % en 30 minutes, sans avoir recours à des procédés complexes et coûteux comme par exemple la réalisation de microgranules hydrosolubles ou de dispersions solides.

La demanderesse a mis en évidence de façon surprenante qu'il est possible de résoudre ce problème grâce à une composition pharmaceutique solide monophasique comprenant un principe actif lipophile, solide et micronisé, un agent tensioactif, un polymère cationique insoluble dans l'eau à pH supérieur ou égal à 5, par exemple de type polyméthacrylate, et un acide organique ou minéral.

La présente Invention concerne aussi le procédé de préparation d'une telle composition.

On connaît des compositions pharmaceutiques à base de principes actifs lipophiles comprenant des polyméthacrylates. Ces formulations sont généralement utilisées pour retarder ou contrôler la libération du principe actif dans l'organisme.

La demande internationale WO 82/01649 décrit une formulation de microgranules à base de fénofibrate comprenant un noyau neutre constitué d'amidon et de saccharose. Le noyau neutre est recouvert d'une solution de fénofibrate avec éventuellement un ou plusieurs excipients et d'une seconde couche microporeuse constituée d'un polymère compatible avec une administration orale, notamment la gomme arabique, les éthers et esters de cellulose, les polyméthacrylates, etc... Cette formulation permet de réaliser un médicament ayant un effet retard contrôlé par le mélange de granules ayant différentes vitesses de libération de fénofibrate. Par exemple, les granules neutres non enrobés de polymère permettent d'obtenir une concentration prédéterminée en fénofibrate.

La demande de brevet FR-A-2 602 423 se rapporte à une composition pharmaceutique comprenant des granules de fénofibrate à libération contrôlée. Chaque granule est constitué d'un noyau inerte, d'une couche de fénofibrate et d'une couche de protection à base de polymères méthacryliques, de PVP, de dérivés de la cellulose ou de polyéthylènes glycols. Dans la couche de base, le fénofibrate est présent sous forme de particules microcristallines de dimension inférieure ou égale à 50 microns (µm). L'utilisation de tels cristaux permet d'obtenir une absorption régulière du fénofibrate contrairement à celle qui est obtenue en suivant l'enseignement de la demande internationale WO 82/01649.

La demande internationale WO 96/36318 se rapporte à une forme pharmaceutique à 3 phases, à libération constante et lente d'un ingrédient actif amorphe pour une administration quotidienne unique. La première phase consiste en un noyau contenant en particulier du fénofibrate amorphe, de la PVP, un éther de cellulose comme excipient et inhibiteur de cristallisation, et un agent tensioactif qui améliore la solubilisation et l'absorption du principe actif amorphe dans le tractus gastro-intestinal. La seconde phase contient un éther de cellulose et un mélange de mono-, di- et triglycérides pour modifier la cinétique de libération du principe actif. La troisième phase est constituée d'un film enrobant faiblement soluble constitué de polymère gastro-résistant, notamment l'Eudragit® L100-55 qui est un copolymère d'acide méthacrylique et d'acrylate d'éthyle (polymère non-cationique).

La demande de brevet FR-A-2 772 615 concerne un comprimé multicouche (bi-couche) pour la libération instantanée puis prolongée de substances actives comprenant au moins deux couches superposées. La première couche permet la libération immédiate du principe actif. La deuxième couche est constituée d'une matrice polymérique poreuse inerte, non biodégradable dans laquelle est dispersée une seconde substance active, pouvant être identique à celle présente dans la première couche. Les substances actives peuvent être notamment hypolipidémiants et la matrice polymérique est par exemple un copolymère non cationique d'acide méthacrylique et d'acrylate d'éthyle ou bien un copolymère cationique tel qu'un copolymère de méthacrylate d'éthylammonium et de méthacrylate d'éthyle (Eudragit® RL, RS, L). Cette seconde couche matricielle est responsable de la libération contrôlée du principe actif.

Enfin, la demande internationale WO 00/72825 traite d'une formulation solide comprenant du fénofibrate ou une statine dispersé dans un polymère hydrophile amorphe. Le fénofibrate est présent dans la formulation sous forme métastable amorphe. Parmi les polymères amorphes sont désignés, entre autres, les polyméthacrylates de la marque Eudragit®. Selon ce document, l'amélioration de la dissolution est espérée car les principes actifs et les polyméthacrylates se présentent sous une forme amorphe. Toutefois, rien ne démontre une amélioration de la dissolution telle qu'elle est annoncée par les auteurs, d'autant plus que les résultats rapportés démontrent une perte de biodisponibilité et un effet retard.

L'ensemble de ces documents démontre que les polymères de type Eudragit® sont généralement utilisés comme film d'enrobage afin de retarder la libération d'un principe actif. Ainsi, les valeurs de dissolution ne peuvent être supérieures à 80 % en 30 minutes.

Selon Lehmann K. *et al., "Acrylic polymers: A review of pharmaceutical applications",* STP Pharma Sciences, 1997, 403-437, les polymères ou copolymères polyméthacryliques, notamment les polymères de type Eudragit® peuvent être utilisés dans les formes pharmaceutiques pour agir sur la libération de principes actifs dans le tractus gastro-intestinal de manière :
- soit immédiate,
- soit retardée (*"lag-time"*),
- soit prolongée.

Les principaux polymères acryliques sont les carbomères (Carbopol®), les copolymères de chlorure de triméthyl ammonioéthyl/méthacrylate (Eudragit® RS/RL), d'acide méthacrylique/méthylméthacrylate (Eudragit® L100, S100, L100-55), de poly(diméthylaminoéthyl)méthacrylate, de méthylméthacrylate et de butylméthacrylate (Eudragit® E100).

Selon la demande de brevet EP 0 436 370, on peut préparer des formulations à libération "pulsée" caractérisées par une période de latence ou *"lag-time"* comprenant un noyau de principe actif et un acide organique, en particulier l'acide succinique, et un film d'enrobage à base d'Eudragit® RS ou RL ayant un groupe ammonium quaternaire. Le film d'enrobage étant légèrement perméable à l'eau, la substance pharmaceutique n'est dissoute ou libérée qu'au bout d'une durée prédéterminée (fonction de l'épaisseur de l'enrobage) mais lorsque le suc digestif a pénétré graduellement dans la préparation et ainsi dissous l'acide organique, la perméabilité du polymère en est augmentée, d'où une dissolution et une libération rapides du principe actif. Le mécanisme probable d'un tel schéma unique de dissolution et de libération du principe actif pourrait s'expliquer par le fait que l'acide organique dissous réagit avec le groupement éthyltriéthylammonium contenu dans le film d'enrobage formé par la dispersion aqueuse et qu'ainsi le polymère très légèrement perméable à l'eau est transformé en un polymère perméable à l'eau.

Les polymères de type Eudragit® comportant un groupe amine tertiaire, en particulier l'Eudragit® E, sont principalement utilisés comme film d'enrobage des formes pharmaceutiques finales pour en masquer le goût. Ces films sont insolubles dans des conditions de pH quasi neutre (pH 5 à 7) du milieu buccal, mais sont très solubles dans un environnement acide comme l'estomac. Ils se dissolvent rapidement à pH inférieur à 5 grâce à une concentration importante des groupes amines tertiaires du monomère N-N-diméthylaminoéthylméthacrylate (50% en poids du polymère). Par contre, ce polymère gonfle à un pH supérieur à 5 à cause des groupes amines très hydrophobes. Ce polymère peut aussi être utilisé dans les formulations à libération lente. En effet, selon Lis P. *et al*., Drug. Dev. Ind. Pharm., 1989, **15,** 1999-2016, quand l'Eudragit® E recouvre des granulés de théophylline, la cinétique de libération est d'ordre zéro.

Dans de rares cas, l'Eudragit® E peut fournir des formes galéniques à libération immédiate. Selon Suzuki H., *et al.,* Chem. Pharm. Bull, 1996, 44, 364-371, le chlorhydrate de bénidipine, qui est un antagoniste calcique, est légèrement soluble dans un acide faible, si bien que sa biodisponibilité est influencée par la variabilité de l'acidité gastrique des patients. Par contre, la molécule est peu soluble dans les solvants organiques généralement utilisés dans les préparations de dispersions solides. Selon le solvant utilisé, les auteurs ont montré que l'Eudragit® E était capable de solubiliser le chlorhydrate de bénidipine grâce à des interactions moléculaires entre le principe actif et le film. Ainsi, des dispersions solides de bénidipine ont pu être préparées et ont montré un taux de dissolution amélioré (pH 6). Toutefois, la bénidipine sous forme de chlorhydrate est une espèce ionisée qui ne se comporte pas comme le fénofibrate. Le fénofibrate n'étant pas un sel, il n'y a pas d'interaction ionique directe possible avec le film d'Eudragit®.

Enfin, certains co-polymères de type acide méthacrylique tels que les Eudragit® peuvent être utilisés pour maintenir un principe actif particulier, la pranidipine, dans un état amorphe afin d'en augmenter la dissolution en solution aqueuse (EP-A-0 410 422).

Ainsi, la présente Invention a pour objet une composition pharmaceutique solide, destinée à l'administration par voie orale, caractérisée par le fait qu'elle contient au sein d'une seule et même phase (phase interne) :
- au moins un principe actif lipophile solide et micronisé,
- au moins un agent tensioactif,
- au moins un polymère cationique insoluble dans l'eau à pH supérieur ou égal à 5, et
- au moins un acide organique ou minéral.

Une telle composition pharmaceutique ne se présente pas sous forme de comprimé multicouche mais sous la forme d'une composition homogène constituée d'une seule phase au sein de laquelle tous les ingrédients décrits ci-dessus sont intimement mélangés. De plus, le polymère cationique insoluble ne confère pas d'effet retard à la composition pharmaceutique.

Selon la présente Invention, le terme "principe actif lipophile" recouvre toute substance lipophile très peu soluble, voire insoluble dans l'eau présentant une activité pharmacologique, thérapeutique etc...

Par "très peu soluble" dans l'eau, on entend selon l'Invention, une solubilité inférieure ou égale à 0,1 mg/ml ou un coefficient de partage (P) octanol/eau, exprimé par le *log P,* supérieur à 2 et de préférence supérieur ou égal à 4. En effet, la lipophilie d'un principe actif (PA) peut être déterminée en fonction de son coefficient de partage (P) entre l'octanol et l'eau qui correspond au rapport concentration du PA dans l'octanol (C_{Oct})/concentration du PA dans l'eau (C_{Eau}). Lorsque le rapport P est supérieur à 1, cela signifie que C_{Oct} est supérieure à C_{Eau} et que par conséquent le PA est lipophile (log *P* > 0). On peut donc en déduire que plus le log *P* d'un PA est élevé et plus celui-ci présente un caractère lipophile prononcé. Ces principes actifs se retrouvent en pratique dans les classes 2 et 4 de la classification biopharmaceutique proposée en 1995 par Amidon *et al*., Pharm. Res., 1995, **12**, 413-420.

Ces principes actifs lipophiles peuvent notamment être choisis parmi les hypolipidémiants, les hormones stéroïdiennes, les antifongiques, les rétinoïdes, les anti-inflammatoires stéroïdiens, les anti-inflammatoires non stéroïdiens (A.I.N.S.), les anti-rétroviraux, les inhibiteurs de protéases ("navirs"), les antiacides, les inhibiteurs de la pompe à protons, les antiémétiques, les vitamines liposolubles, les médicaments du système cardiovasculaire, les antiagrégants plaquettaires, les anticancéreux, certains extraits végétaux et leurs principes actifs isolés ou dérivés, les immunosuppresseurs, les médicaments du système nerveux central, les antimigraineux, les antibiotiques et les antiparasitaires.

Parmi les hypolipidémiants, on peut notamment citer les fibrates comme le 1-méthyléthyl ester de l'acide 2-(4-(4-chlorobenzoyl)phénoxy)-2-méthyl-propanoïque également dénommé fénofibrate et les produits apparentés de la classe des fibrates tels que le bézafibrate, le ciprofibrate et le gemfibrozil.

On peut également citer, parmi les autres hypolipidémiants, les bisthioéthers, dont le probucol et le tiadénol, la classe des inhibiteurs de la HMG Co-A réductase (statines) tels que par exemple la simvastatine, la mévastatine, la lovastatine, l'atorvastatine, la pravastatine, la fluvastatine, la cerivastatine, ainsi que la classe des inhibiteurs de l'ACAT tels que le mélinamide et ses analogues structuraux.

A titre d'hormones stéroïdiennes, on peut notamment citer les oestrogènes dérivés et esters de l'estradiol, la progestérone, le danazol, la testostérone et les esters et dérivés de testostérone. On peut également citer les anti-androgènes dont le flutamide, la nilutamide ; les inhibiteurs de la 5 α-réductase, les inhibiteurs compétitifs de la testostérone tels que le finastéride ; les dérivés de la quinazoline tels que l'alfuzosine ; les agonistes/antagonistes non stéroïdiens des récepteurs oestrogéniques tels que le tamoxifène et le raloxifène.

Parmi les antifongiques, on peut notamment citer les antifongiques azolés (conazoles) dont l'itraconazole, le miconazole, le kétoconazole et le fluconazole, ainsi que la griséofulvine, l'amphotéricine B et la terbinafine.

Parmi les rétinoïdes, on peut citer, en premier lieu, les rétinoïdes dérivés de la vitamine A comme la trétinoïne, encore connue sous le nom d'acide tout-trans rétinoïque ou d'acide tout-trans vitamine A, l'isotrétinoïne qui correspond à l'isomère 13-cis de la trétinoïne, et qui, de ce fait, est également appelée acide 13-cis rétinoïque ou acide 13-cis vitamine A, l'acide 9-cis rétinoïque ou acide 9-cis vitamine A, l'acitrétine, l'étrétinate, mais également les rétinoïdes acétyléniques comme le tazarotène, les rétinoïdes dérivés du naphtalène comme le lonapalène et l'acide 2-(5,6,+,8-tétrahydrométhyl-2-anthryl)-4-thiophénocarboxylique, et les rétinoïdes à cycle adamantyle tels que l'adapalène, l'acide 6-[3-(1-adamantyl)-4-hydroxyphényl]-2-naphtoïque et l'acide 4-[3-(1-adamantyl)-4-méthoxybenzamido]-benzoïque et leurs esters.

A titre d'anti-inflammatoires stéroïdiens, on peut notamment citer les glucocorticoïdes tels que la prednisolone, la cortisone, ses esters et dérivés.

A titre d'A.I.N.S., on peut notamment citer l'acide méfénamique, le naproxène, le nabumétone, l'ibuprofène et les inhibiteurs de la COX-2 tels que le celecoxib, le rofecoxib, le parecoxib et le valdecoxib.

Les antirétroviraux et les inhibiteurs des protéases sont des composés très peu solubles dans l'eau, dont les coefficients de partage peuvent être calculés ou déterminés par voie analytique, parmi lesquels on peut citer l'amprenavir (solubilité 0,04 mg/l), le saquinavir et le saquinavir mésylate (solubilité 2,22 mg/ml) et le ritonavir (presque insoluble dans l'eau).

A titre d'antiacides et d'inhibiteurs de la pompe à protons, on peut notamment citer l'oméprazole, le pantoprazole, le rabéprazole (ou pariprazole), la lansoprazole et le timoprazole.

A titre d'antiémétiques, on peut notamment citer la dompéridone, les antagonistes de la sérotonine ("setrons") tels que l'ondansetron, le granisetron et l'azasetron.

A titre de vitamines liposolubles, on peut notamment citer les vitamines A ou rétinol, D dont le calcitriol, E ou tocophérols, K ou ménadione.

Parmi les médicaments du système cardiovasculaire, on peut notamment citer les antagonistes de l'angiotensine II (sartans) tels que le valsartan, le losartan, l'irbésartan, le candesartan, le tasosartan, le telmisartan ; les α- et β-bloquants tels que le carvédilol, le celiprolol ; les inhibiteurs calciques (dihydropyridines) tels que le verapamil, le diltiazem, la nifédipine et la nitrendipine. On peut également citer d'autres composés, anti-hypertenseurs, tels que les peptides inhibiteurs de la rénine, les dérivés d'oxazolidinone ou glycol peptides substitués par des restes aminés et/ou des noyaux hétérocycliques azolés ou thiazolés.

A titre d'antiagrégants plaquettaires, on peut notamment citer la ticlopidine et l'hydrogénosulfate de clopidogrel ; les anticoagulants coumariniques dont la warfarine et les composés du groupe de l'indan-dione, dont la phényl indan-dione.

A titre d'anticancéreux, on peut notamment citer le paclitaxel et le docétaxel qui sont des composés insolubles dans l'eau ; les extraits et alcaloïdes de *Vinca minor* tels que la vincristine, la vincaleucoblastine ou vinblastine, la vincamine et leurs dérivés ; les alcaloïdes *d'Ochrosia elliptica* dont l'ellipticine.

Parmi les extraits végétaux et leurs principes actifs isolés ou dérivés, on peut notamment citer les alcaloïdes tels que la yohimbine, les flavonoïdes dont la diosmine, la rutine et ses dérivés tels que la troxérutine ; les extraits de *Pygeum africanum* ou de *Serenoa repens.*

A titre d'immunosuppresseurs, on peut en particulier citer la ciclosporine et le tacrolimus.

Parmi les divers médicaments du système nerveux central, figurent les tranquillisants, les sédatifs, les hypnotiques et anesthésiques. A titre d'exemple, on peut citer les barbituriques tels que les thiobarbituriques ; les anxiolytiques tels que les benzodiazépines ; les antihistaminiques tels que la terfénadine, la loratadine, la desloratadine et la cétirizine ; les antidépresseurs tricycliques et sérotoninergiques tels que la fluoxétine, la paroxétine, la sertraline et le citalopram.

Parmi les antimigraineux, on peut citer les composés du groupe des "triptans" sérotoninergiques tels que l'oxitriptan, le sumatriptan et l'almotriptan.

Parmi les antibiotiques, on peut notamment citer les céphalosporines de troisième génération telles que le céfixime trihydrate et le cefpodoxime proxétil ; les macrolides tels que l'azithromycine, la clarithromycine, la roxithromycine, la josamycine, la spiramycine ; les synergistines telles que la pristinamycine ; les quinolones et les quinoxalines, dont le carbadox.

Parmi les antiparasitaires on peut citer les antipaludiques tels que l'halofantrine, la méfloquine, le proguanil, la pyriméthamine, les extraits *d'Artemisia spp* et les substances isolées de ces extraits et leurs dérivés tels que l'artémisine, l'artémisinine ; la série des avermectines, dont l'ivermectine pratiquement insoluble dans l'eau ; les anthelminthiques dérivés du benzimidazole à usage vétérinaire tels que par exemple le tiabendazole, l'albendazole, le mébendazole, le fenbendazole et le triclabendazole ; la classe des salicylanilides à usage vétérinaire, utilisés dans les fascioloses (douvicides) et autres parasitoses comprenant notamment le bromoxanide, le brotianide, le clioxanide, le closantel, l'oxyclozanide, le rafoxanide ainsi que le dibromosalan et le tribromosalan.

Selon l'Invention, le ou les principes actifs sont de préférence choisis parmi les hypolipidémiants parmi lesquels le fénofibrate est tout particulièrement préféré, les hormones stéroïdiennes parmi lesquelles la progestérone est tout particulièrement préférée et les antifongiques parmi lesquels l'itraconazole est tout particulièrement préféré.

Le ou les principes actifs représentent de préférence de 10 à 90 % en poids du poids total de la composition pharmaceutique, ces pourcentages étant bien évidemment modulés selon les besoins de la formulation eu égard aux doses efficaces du principe actif considéré.

Selon une forme de réalisation avantageuse de l'Invention, le principe actif micronisé se présente, avant son mélange avec les autres constituants de la présente composition pharmaceutique, sous la forme d'une poudre dont les particules ont une dimension homogène de taille inférieure ou égale à 45 µm et, si besoin, inférieure ou égale à 10 µm.

Ainsi qu'on l'a vu précédemment, la présence d'un agent tensioactif est l'une des caractéristiques essentielles de la composition pharmaceutique conforme à l'Invention.

Cet agent tensioactif est de préférence choisi parmi les composés présentant une valeur de la balance hydrophile-lipophile (HLB) élevée, de préférence supérieure ou égale à 15. Parmi de tels agents tensioactifs, on peut en particulier citer le laurylsulfate de sodium (HLB 40), les poloxamers (HLB 16-29), les macrogol éthers d'alcools organiques (HLB 15-18), et les sucrose-esters d'acides organiques (HLB 15-16).

Selon un mode de réalisation préféré de l'Invention, l'agent tensioactif est le laurylsulfate de sodium.

Le ou les agents tensioactifs utilisables dans la composition pharmaceutique conforme à l'Invention représentent de préférence de 1 à 10 % en poids du poids total de ladite composition et encore plus préférentiellement de 5 à 10 % en poids.

Selon une forme de réalisation particulière de l'Invention, l'agent tensioactif se présente sous forme solide. Dans ce cas, il peut être co-micronisé avec le principe actif. Les techniques de micronisation de principes actifs (en présence ou non d'un agent tensioactif solide) sont bien connues de l'homme de l'art et peuvent notamment être réalisées au moyen de broyeurs à jet d'air ou de broyeurs à jet liquide (Microfluidizer®). On peut notamment utiliser un principe actif co-micronisé avec du laurylsulfate de sodium.

Le rapport pondéral principe actif/agent tensioactif est de préférence compris entre 100/1 et 5/1.

Parmi les polymères cationiques insolubles dans l'eau à pH supérieur ou égal à 5 utilisables conformément à la présente Invention, on peut citer les polymères acryliques contenant un groupe amine tertiaire tels que les polymères de type méthacrylate aminoalkyle solubles en milieu acide, c'est à dire à pH inférieur à 5. Parmi ces polymères, on peut par exemple citer le terpolymère de poly(diméthylaminoéthyl)méthacrylate, de méthyl méthacrylate et de butyl méthacrylate et le terpolymère de poly(diéthylaminoéthyl)méthacrylate, de méthyl méthacrylate et de butyl méthacrylate.

Selon l'Invention, le polymère cationique préféré est un terpolymère de poly(diméthylaminoéthyl)méthacrylate, de méthyl méthacrylate et de butyl méthacrylate tel que celui vendu sous la dénomination Eudragit® E par la société Röhm Pharma.

Selon l'Invention, le ou les polymères cationiques insolubles représentent de préférence de 0,5 à 15 % en poids par rapport au poids total de la composition pharmaceutique et encore plus particulièrement de 1 à 10 %.

Par ailleurs, selon un mode de réalisation préféré de la composition pharmaceutique conforme à l'Invention, le rapport pondéral polymère cationique insoluble / principe actif est compris entre 1/5 et 1/30.

L'acide organique ou minéral présent dans la composition pharmaceutique conforme à l'Invention peut notamment être choisi parmi l'acide citrique, l'acide succinique, l'acide fumarique, l'acide acétique, l'acide phosphorique, l'acide sulfurique et l'acide chlorhydrique.

L'utilisation d'acide succinique est particulièrement préférée selon l'Invention.

Cet acide organique ou minéral représente de préférence de 1 à 10 % en poids par rapport au poids total de la composition pharmaceutique conforme à l'Invention, et encore plus préférentiellement de 2 à 7 % en poids.

L'utilisation d'un acide organique ou minéral permet de créer un micro-pH favorisant la solubilisation du polymère cationique insoluble dans l'eau à pH supérieur ou égal à 5. En effet, le polymère devient soluble dans l'eau en formant un sel en présence de restes acides.

Selon une forme de réalisation préférée de l'Invention, le rapport pondéral acide organique ou minéral/polymère cationique insoluble dont dépend aussi, de façon surprenante, la dissolution du principe actif, est compris entre 6/1 et 0,25/1 et encore plus préférentiellement entre 2/1 et 0,5/1.

Par ailleurs, le ratio principe actif lipophile/acide minéral ou organique au sein de la composition pharmaceutique conforme à l'Invention est de préférence compris entre 1/1 et 30/1 et encore plus préférentiellement, il est égal à 20/1.

Une composition pharmaceutique particulièrement préférée selon l'Invention comprend (en poids par rapport au poids total de ladite composition) :
- 40 à 80 % environ de fénofibrate à titre de principe actif lipophile,
- 2 à 10 % environ d'agent tensioactif,
- 2 à 10 % environ d'un terpolymère de poly(diméthylaminoéthyl)-méthacrylate, de méthyl méthacrylate et de butyl méthacrylate (Eudragit E ®), et
- de 2,5 à 5 % environ d'un acide minéral ou organique, la teneur de 5 % environ étant particulièrement préférée.

Par ailleurs, la composition pharmaceutique particulièrement préférée et telle que décrite ci-dessus peut être diluée si besoin par incorporation de divers agents d'écoulement des poudres et ce, dans une proportion voisine de 40 à 50 %.

La composition pharmaceutique conforme à l'Invention et telle que décrite de façon générale ci-dessus permet d'améliorer la dissolution des principes actifs lipophiles très peu solubles, voire insolubles dans l'eau, et en particulier celle du fénofibrate et d'accroître ainsi leur absorption intestinale. L'Invention fournit donc une composition pharmaceutique comprenant un principe actif lipophile présentant, dans le cas particulier du fénofibrate, une dissolution d'au moins 50 % en 15 minutes, supérieure à 80 % en 30 minutes, supérieure à 85 % en 45 minutes et supérieure à 90 % en 60 minutes, telle que mesurée conformément à la méthode de la palette tournante à 75 tours/min selon la Pharmacopée Européenne, dans un milieu de dissolution constitué de laurylsulfate de sodium 0,1 M en solution aqueuse portée à 37°C.

Sans vouloir être lié à une quelconque théorie, l'amélioration de la dissolution des principes actifs lipophiles et en particulier de celle du fénofibrate pourrait être facilitée par le micro-pH acide créé par l'acide organique ou minéral au contact du polymère cationique insoluble dans l'eau, la dissolution de celui-ci entraînant une meilleure mouillabilité de l'agent tensioactif d'où une amélioration de la dissolution du principe actif.

La phase interne de la composition pharmaceutique conforme à l'Invention peut également renfermer, selon les besoins de la formulation, un ou plusieurs excipients choisis parmi les agents diluants et/ou liants, les agents désintégrants et les adjuvants classiques de pulvérisation, de compression, de lubrification et d'écoulement des poudres connus de l'homme de l'art, à condition, toutefois, que la présence d'un tel excipient ne dénature pas le caractère homogène et monophasique de ladite phase interne.

Parmi de tels excipients, on peut plus particulièrement citer les agents d'écoulement des poudres tels que le talc, la silice, l'acide stéarique, le stéarate de magnésium et ses dérivés, les agents liants tels que les celluloses telles que la cellulose microcristalline, les dextrines et maltodextrines, et les agents désintégrants tels que l'amidon modifié comme le carboxyméthyl amidon vendu par exemple sous la dénomination Primogel®, et leurs mélanges.

Lorsqu'ils sont utilisés dans la phase interne de la composition pharmaceutique conforme à l'Invention, les agents d'écoulement des poudres représentent alors de préférence de 40 à 50 % en poids environ par rapport au poids total de la composition pharmaceutique conforme à l'Invention.

La composition pharmaceutique conforme à la présente Invention et telle que décrite précédemment peut être incorporée, parmi d'autres ingrédients de formulation, dans la mise en forme d'un produit fini médicamenteux. La forme pharmaceutique finale ainsi obtenue comporte, outre la composition pharmaceutique décrite ci-dessus, d'autres composants constituant une phase externe comprenant un ou plusieurs excipients.

Dans le cadre de la présente Invention, on entend par "phase externe", tout revêtement présent sur la composition pharmaceutique telle que décrite précédemment, sur un produit semi-ouvré, sur le principe actif modifié ou sur un produit intermédiaire. Selon l'Invention, on entend par "produit semi-ouvré" ou par principe actif modifié ou encore par produit intermédiaire, toute composition intermédiaire entre la matière première constituée par le principe actif lipophile micronisé et le produit fini. Ce dernier correspond au médicament sous une forme galénique achevée telle que comprimé, gélule ou encore sachet. Ce produit semi-ouvré peut se présenter sous différentes formes physiques, en particulier atomisat ou granulé, avant d'être incorporé dans la forme galénique finale.

Cette phase externe peut notamment comprendre un ou plusieurs excipients classiques tels que ceux utilisés dans le domaine pharmaceutique et chimiquement compatibles avec le principe actif mis en oeuvre conformément à l'Invention.

Parmi de tels excipients, on peut notamment citer les agents diluants et/ou liants, les agents de désintégration, les charges, les pigments, les agents mouillants, les tampons, ainsi qu'un ou plusieurs adjuvants susceptibles de faciliter l'écoulement des poudres tels que les agents d'écoulement ou lubrifiants (talc, silice, acide stéarique, le stéarate de magnésium et ses dérivés), couramment employés pour améliorer les caractéristiques rhéologiques des produits pulvérulents et leur mise en forme pharmaceutique finale (ensachage, répartition en gélules, compression).

Parmi les agents liants pouvant être utilisés dans la composition pharmaceutique conforme à l'Invention, on peut notamment citer les celluloses telles que la cellulose microcristalline, les dextrines, les maltodextrines, les cyclodextrines, le mannitol et leurs mélanges. Les agents liants facilitent les opérations de compressions nécessaires à l'obtention de comprimés et confèrent une dureté adéquate à ces derniers. Lorsqu'ils sont utilisés, ces agents liants représentent de préférence de 5 à 60% du poids total de la formulation galénique finale ou forme pharmaceutique.

Parmi les agents de désintégration pouvant être utilisés dans la composition pharmaceutique conforme à l'Invention, on peut notamment citer la carboxyméthylcellulose de sodium réticulée (croscarmellose sodique), l'amidon modifié tel que le carboxyméthyl amidon vendu par exemple sous la dénomination Primogel® et leurs mélanges.

Parmi les agents d'écoulement ou lubrifiants pouvant être utilisés dans la composition pharmaceutique conforme à l'Invention, on peut notamment citer la silice colloïdale anhydre telle que le produit vendu sous la dénomination Aerosil®, le dioxyde de silice précipitée, le talc, le stéarate de magnésium, l'acide stéarique, le polyéthylèneglycol (PEG), et leurs mélanges ; la silice colloïdale anhydre étant particulièrement préférée. Ces agents d'écoulement sont utilisés pour empêcher les composants des comprimés ou des granulés de former des agrégats au cours de la préparation de ces comprimés ou granulés et pour réduire également les frictions pendant les opérations de compression. Lorsqu'ils sont utilisés, ils sont généralement présents dans des proportions comprises entre 0,1 et 3 % du poids total de la forme galénique finale.

La phase externe peut également renfermer un acide organique ou minéral tels ceux définis ci-dessus.

Lorsqu'elle est présente, la phase externe peut représenter jusqu'à 60 % en poids du poids total de la forme galénique finale, de préférence jusqu'à 45 %.

Selon un mode de réalisation préféré, la composition pharmaceutique selon la présente Invention se présente sous forme de comprimés.

Selon un autre mode de réalisation, la composition pharmaceutique selon la présente Invention se présente sous forme de granulés répartis en gélules.

La présente Invention a également pour objet un procédé de préparation d'une telle composition pharmaceutique comprenant les étapes suivantes :
a) le mélange d'au moins un principe actif lipophile solide et micronisé, d'au moins un agent tensioactif, d'au moins un polymère cationique insoluble dans l'eau à pH supérieur ou égal à 5 et d'au moins un acide organique ou minéral,
b) la granulation ou l'atomisation du mélange obtenu ci-dessus à l'étape a),
c) l'addition éventuelle d'une phase externe comprenant un ou plusieurs excipients, puis
d) la compression ou la répartition en gélules du mélange obtenu à l'issue de l'étape b) ou c) lorsque cette dernière est réalisée.

Lors de l'étape a), le mélange peut en outre éventuellement renfermer un agent d'écoulement des poudres tels que ceux décrits précédemment.

Selon une forme de réalisation particulière de l'Invention, le procédé conforme à la présente Invention peut comprendre en outre une étape préliminaire de co-micronisation du principe actif avec le ou les agents tensioactifs.

Selon une première variante du procédé conforme à l'Invention, l'étape b) est réalisée par granulation, de préférence en lit d'air fluidisé.

Selon cette première variante, le mélange des constituants lors de l'étape a) comprend de préférence les sous-étapes suivantes :
a1) la préparation d'une solution ou d'une suspension comprenant au moins un acide organique ou minéral et au moins un polymère cationique insoluble dans l'eau à pH supérieur ou égal à 5, dans un liquide de granulation,
a2) la pulvérisation du mélange préparé ci-dessus à l'étape a1) sur le principe actif micronisé et préalablement mélangé à l'agent tensioactif solide ou co-micronisé avec ledit principe actif, à une température compatible avec la stabilité physique des substances engagées dans la formulation, de préférence comprise entre 20 et 45°C °C, et tout particulièrement comprise entre 30 et 40°C,
a3) la récupération des granulés fluidisés ainsi obtenus,
a4) le calibrage, par exemple par tamisage, des granulés fluidisés,
a5) le séchage des granulés fluidisés.

Le liquide de granulation utilisé lors de la sous-étape a1) peut être choisi parmi l'eau, les solvants organiques et leurs mélanges en toutes proportions, ledit liquide pouvant être éventuellement additionné d'un tampon spécifié par les Pharmacopées en vigueur, ledit tampon présentant alors de préférence un pH compris entre 2 et 5.

Selon une forme de réalisation préférée, le liquide de granulation est un solvant organique ou un mélange de solvants organiques aptes à permettre la dissolution du polymère cationique utilisé. A titre de tels solvants on peut notamment citer les alcools inférieurs tels que l'éthanol et le propanol, l'acétone et leurs mélanges.

Lorsque le polymère cationique est un terpolymère de poly(diméthylaminoéthyl)méthacrylate, de méthyl méthacrylate et de butyl méthacrylate (Eudragit® E), alors un liquide de granulation particulièrement préféré selon l'Invention est un mélange propanol/acétone, de préférence en proportions 60/40 (v/v).

Selon cette première variante, le ou les polymères cationiques et l'acide minéral ou organique utilisés représentent, dans leur ensemble, de préférence de 10 à 15 % en poids du poids total du liquide de granulation.

Lorsque l'agent tensioactif ne se présente pas sous la forme d'un co-micronisat avec le principe actif, ledit principe actif micronisé et l'agent tensioactif solide utilisés lors de l'étape a2) ci-dessus sont de préférence pré mélangés à l'aide d'un mélangeur à poudres tel qu'un mélangeur par retournement à cuve fixe ou mobile ou un mélangeur à outils mobiles tel qu'un mélangeur planétaire, à vis, à socs ou à rubans.

Selon une deuxième variante du procédé conforme à l'Invention, l'étape b) est réalisée par atomisation.

Selon cette deuxième variante, le mélange des constituants lors de l'étape a) comprend de préférence les sous-étapes suivantes :
a'1) la préparation d'une solution acide ou tampon contenant un acide minéral ou organique et une base forte, ladite solution présentant un pH inférieur à 5 et de préférence compris entre 2 et 5,
a'2) la préparation d'une suspension par addition à cette solution tampon d'au moins un principe actif lipophile solide et micronisé, d'au moins un agent tensioactif, éventuellement co-micronisé avec ledit principe actif et d'au moins un polymère cationique insoluble dans l'eau à pH supérieur ou égal à 5, sous agitation,
a'3) l'atomisation de ladite suspension,
a'4) la récupération du produit atomisé.

Lors de l'étape a'1), la solution tampon peut notamment être constituée d'un mélange d'eau, d'acide succinique et de soude.

L'étape a'2) est de préférence réalisée sous agitation magnétique et homogénéisation pendant 10 à 15 minutes, à une vitesse comprise entre 8 000 et 10 000 tours/minute. Il doit être cependant bien entendu que la transposition d'échelle pour une production industrielle requiert l'adaptation en conséquence des paramètres de durée et de vitesse de mélange puis d'homogénéisation, en fonction de l'équipement employé. Ces paramètres seront facilement ajustés par l'homme de l'art sans sortir du cadre ni de la portée de la présente Invention.

Selon une forme particulière de réalisation de cette deuxième variante, les étapes a'1) et a'2) peuvent être réalisées simultanément.

Lors de l'étape a'2) et selon une variante de ce procédé, une fraction des composants de la phase externe lorsque celle-ci est utilisée, notamment les agents favorisant l'écoulement des poudres tels que l'amidon, la silice et/ou le talc, peut être introduite dans la suspension à atomiser.

L"atomisation, est un procédé bien connu de l'homme de l'art qui a longtemps été réservée au domaine de l'agroalimentaire. L'atomisation consiste à réduire un corps en fines particules, à partir de son état liquide. Il s'agit donc d'atomiser une solution ou une suspension d'un ou de plusieurs solides *via* une buse ou un autre système (par exemple, turbine) et ensuite d'évaporer le solvant des gouttelettes formées.

Selon l'Invention, l'étape a'3) d'atomisation est de préférence réalisée à l'aide d'une buse bi-fluide, d'une buse haute pression ou d'une turbine, avec un débit compris entre 3 et 7 kg/h, de préférence 5 kg/h environ.

La nature de la poudre résultante est fonction de plusieurs variables, notamment la concentration initiale du soluté, la répartition de la taille des gouttelettes obtenues et le taux de solvant extrait.

D'une manière générale, les particules de poudre atomisée sont homogènes, sphériques, de taille uniforme et de faible densité avec une dissolution rapide.

L'homogénéité de la taille des particules et leur forme sphérique donnent des poudres présentant un bon écoulement et donc directement compressibles.

Selon l'Invention l'étape d) de compression peut par exemple être réalisée par mélange à sec et compression directe, en présence ou non d'une phase externe telle que décrite précédemment.

Selon un mode de réalisation de l'Invention particulièrement avantageux, une forme pharmaceutique finale peut être préparée selon un procédé comprenant les étapes suivantes :
- la préparation d'un produit semi-ouvré à base de fénofibrate sous forme atomisée, selon la deuxième variante du procédé tel que décrit ci-dessus,
- le mélange du produit atomisé avec une phase externe, et
- la compression dudit mélange ou sa répartition en gélules.

La forme pharmaceutique ainsi préparée permet d'améliorer la dissolution du fénofibrate et d'accroître ainsi son absorption intestinale. L'Invention fournit donc un procédé de préparation d'une composition pharmaceutique sous forme de produit intermédiaire séché par atomisation comprenant un principe actif fénofibrate présentant une dissolution d'au moins 50 % en 15 minutes, supérieure à 80 % en 30 minutes, à 85 % en 45 minutes et supérieure à 90 % en 60 minutes, telle que mesurée conformément à la méthode de la palette tournante à 75t/min selon la Pharmacopée Européenne, dans un milieu de dissolution constitué de laurylsulfate de sodium 0,1 M en solution aqueuse portée à 37°C.

Outre les dispositions qui précèdent, l'Invention comprend encore d'autres dispositions qui ressortiront de la description qui va suivre, qui se réfère à des exemples de préparation de compositions pharmaceutiques conformes à l'Invention à base de fénofibrate ainsi qu'à un exemple décrivant une étude comparant la vitesse de dissolution de compositions pharmaceutiques conformes à l'Invention à celle de la spécialité commerciale Lipanthyl® 160 mg.

Il doit être bien entendu toutefois que ces exemples sont donnés uniquement à titre d'illustration de l'objet de l'Invention, dont ils ne constituent en aucune manière une limitation.

### EXEMPLE 1 : PRÉPARATION DE COMPOSITIONS PHARMACEUTIQUES A BASE DE FÉNOFIBRATE PAR ATOMISATION

On prépare une solution tampon succinate pH 4,6 par mélange de 23,6 g d'acide succinique, 164 ml de soude 1 N dans de l'eau en quantité suffisante pour avoir 2 litres de solution tampon. A cette solution, on ajoute ensuite 8 g de laurylsulfate de sodium (LSNa).

On ajoute ensuite, sous agitation magnétique et homogénéisation pendant 10 minutes à 10 000 tours/min à l'aide d'un mélangeur/homogénéisateur TURRAX®, 400 g de fénofibrate micronisé et 40 g d'un polymère insoluble dans l'eau à pH supérieur ou égal à 5 : copolymère cationique d'acide méthacrylique ayant un groupement diméthylaminoéthyl vendu sous la dénomination EUDRAGIT® E 100 ou EUDRAGIT® E PO.

On obtient une suspension qui est ensuite atomisée dans une chambre d'atomisation équipée d'une buse bi-fluide avec un débit de 5 kg/h.

Le produit ainsi atomisé (fénofibrate modifié : 195 mg) est ensuite mélangé avec une phase externe dont la composition est indiquée dans le Tableau I ci-après :

**TABLEAU I**

| | **FORMULATIONS** | | | | |
|---|---|---|---|---|---|
| **Excipients (en mg)** | **A** | **B** | **C** | **D** | **E** |
| Cellulose microcristalline | 200 | 200 | 200 | 225 | 225 |
| Crospovidone | 42 | - | - | - | - |
| Croscarmellose sodique | - | 42 | 42 | 42 | - |
| Carboxyméthyl amidon sodique | - | - | - | - | 42 |
| PEG 6000 | 3 | 50 | - | - | - |
| Stéarate de magnésium | 50 | - | 50 | 25 | 25 |
| Silice colloïdale anhydre | - | 3 | 3 | 3 | 3 |

Le mélange ainsi obtenu est ensuite comprimé pour conduire à des formulations galéniques finales A à E sous forme de comprimés renfermant chacun 195 mg de fénofibrate modifié correspondant à 160 mg de fénofibrate.

### EXEMPLE 2 : PRÉPARATION D'UNE FORMULATION PHARMACEUTIQUE A BASE DE FÉNOFIBRATE PAR GRANULATION LAF

A l'aide d'un mélangeur à poudre, on mélange 8 g de LSNa, 400 g de fénofibrate micronisé et 24 g d'acide succinique.

Ce mélange est ensuite granulé en lit d'air fluidisé en présence d'un polymère cationique insoluble dans l'eau à pH supérieur ou égal à 5 (Eudragit® E) à 12,5 g dans 100 g d'un mélange propanol/acétone 60/40 (v/v).

Le produit de granulation (fénofibrate modifié : 195 mg) est ensuite mélangé avec une phase externe dans les proportions indiquées ci-après dans le Tableau II, pour conduire à la formulation F :

**TABLEAU II**

| **Excipients (en mg)** | **Formulation F** |
|---|---|
| Cellulose microcristalline | 225 |
| Carboxyméthyl amidon sodique | 42 |
| Stéarate de magnésium | 25 |
| Silice colloïdale anhydre | 3 |

La formulation F est ensuite directement comprimée pour conduire à des comprimés F renfermant chacun 195 mg de fénofibrate modifié, correspondant à 160 mg de fénofibrate.

### EXEMPLE 3 : ETUDE COMPARATIVE DES PROFILS DE DISSOLUTION DES FORMULATIONS A à F COMPARATIVEMENT A CELUI DU LIPANTHYL® 160 MG

Les comprimés commercialisés sous la dénomination Lipanthyl® 160 mg renferment 160 mg de fénofibrate micronisé ainsi qu'un mélange d'excipients composés de LSNa, de lactose monohydrate, de polyvidone, de cellulose microcristalline, de silice colloïdale anhydre, de crospovidone et de stéaryle fumarate de sodium. Ces comprimés correspondent à la composition pharmaceutique telle que décrite également dans la demande de brevet FR-A-2 758 459.

Cette étude de dissolution consiste à mesurer la vitesse de dissolution des comprimés testés selon la méthode de la palette tournante, à 75 tours/min selon la Pharmacopée Européenne (4^{ème} édition, paragraphe 2.9.3.), dans un milieu de dissolution constitué de LSNa 0,1 M à 37°C.

Pour chaque comprimé, la dissolution a été mesurée à 15, 30, 45 et 60 minutes.

Les résultats obtenus figurent dans le tableau III ci-après (les valeurs indiquées sont les moyennes de trois mesures des % de dissolution) :

**TABLEAU III**

| **FORMULATIONS** | **TEMPS (en minutes)** | | | |
|---|---|---|---|---|
| | **15** | **30** | **45** | **60** |
| **A** | 57 | 84 | 89 | 91 |
| **B** | 78 | 86 | 89 | 89 |
| **C** | 78 | 86 | 89 | 90 |
| **D** | 88 | 95 | 95 | 100 |
| **E** | 84 | 92 | 94 | 96 |
| **F** | 83 | 87 | 90 | 93 |
| **Lipanthyl® 160 mg** | 75 | 100 | 100 | 100 |

Ces résultats montrent que les formulations conformes à l'Invention permettent d'atteindre des taux de dissolution d'au moins 50 % en 15 minutes, supérieurs à 80 % en 30 minutes, à 85 % en 45 minutes et supérieurs à 90 % en 60 minutes.

Par conséquent, les formulations conformes à la présente Invention, tout en étant beaucoup plus simples et moins coûteuses à préparer que les compositions décrites dans la demande de brevet FR-A-2 758 459, permettent d'atteindre des taux de dissolution tout à fait satisfaisants et comparables à celui obtenu avec le Lipanthyl® 160 mg.

### EXEMPLE 4 : PREPARATION DE COMPOSITIONS PHARMACEUTIQUES A BASE DE FENOFIBRATE PAR ATOMISATION

On prépare une solution acide ayant un pH compris entre 2 et 5, par mélange de 1 kg d'acide succinique dans 201 d'eau. A cette solution, on ajoute ensuite 3 kg de LSNa.

Parallèlement, on prépare une solution à base d'amidon et de silice par ajout de 13 à 15 kg d'amidon modifié lipophile vendu sous la dénomination Amidon EmCap® par la société Cerestar et de 1 à 2,5 kg de silice colloïdale vendue sous la dénomination Tixosil® par la société Rhodia dans 401 d'eau.

On ajoute ensuite, sous agitation magnétique et homogénéisation pendant 10 minutes à 10 000 tours/min à l'aide d'un mélangeur planétaire, 15 kg de fénofibrate micronisé, 0,5 à 1 kg d'un polymère insoluble vendu sous la dénomination EUDRAGIT® E PO ainsi que 50 kg environ de la solution à base d'amidon et de silice.

On obtient ensuite une suspension qui est ensuite atomisée comme décrit ci-dessus à l'exemple 1.

Le produit ainsi atomisé (fénofibrate modifié) est ensuite incorporé dans la formule à raison de 55 à 65 % (soit environ 350 à 400 mg, selon le titre en fénofibrate), dans le mélange dit de phase externe dont la composition est indiquée dans le Tableau IV ci-après :

**TABLEAU IV**

| | **FORMULATIONS** |
|---|---|
| **Excipients (en mg)** | **G** |
| Mannitol | 90 |
| Cellulose microcristalline | 80-90 |
| Croscarmellose sodique | 42 |
| Stéarate de magnésium | 2 à 2,5 |
| Silice colloïdale anhydre | 3 à 6,5 |

Le mélange ainsi obtenu est ensuite comprimé pour conduire à une formulation galénique finales G sous forme de comprimés renfermant chacun de 350 à 400 mg de fénofibrate modifié correspondant à 160 mg de fénofibrate.

### EXEMPLE 5 : ETUDE COMPARATIVE DES PROFILS DE DISSOLUTION DE LA FORMULATION G COMPARATIVEMENT A CELUI DU LIPANTHYL® 160 MG

Cette étude de dissolution a été réalisée en utilisant les comprimés commercialisés sous la dénomination Lipanthyl® 160 mg ainsi que la composition G préparée ci-dessus à l'exemple 4. L'étude de la vitesse de dissolution a été effectuée selon la méthode décrite ci-dessus à l'exemple 3.

Pour chaque comprimé, la dissolution a été mesurée à 15, 30, 45 et 60 minutes.

Les résultats obtenus figurent dans le tableau V ci-après (les valeurs indiquées sont les moyennes de trois mesures des % de dissolution) :

**TABLEAU V**

| **FORMULATIONS** | **TEMPS (en minutes)** | | | |
|---|---|---|---|---|
| | **15** | **30** | **45** | **60** |
| **G** | 77 | 98 | 99 | 99 |
| **Lipanthyl® 160 mg** | 75 | 100 | 100 | 100 |

Tout comme dans l'exemple 3, ces résultats montrent que la formulation conforme à l'Invention permet d'atteindre des taux de dissolution d'au moins 50 % en 15 minutes, supérieurs à 80 % en 30 minutes, à 85 % en 45 minutes et supérieurs à 90 % en 60 minutes.

## Revendications

1. Composition pharmaceutique solide, destinée à l'administration par voie orale, **caractérisée par le fait qu'**elle contient au sein d'une seule et même phase (phase interne) :
- au moins un principe actif lipophile solide et micronisé,
- au moins un agent tensioactif,
- au moins un polymère cationique insoluble dans l'eau à pH supérieur ou égal à 5, et
- au moins un acide organique ou minéral.

2. Composition selon la revendication 1, **caractérisée par le fait que** le principe actif lipophile est choisi parmi les hypolipidémiants, les hormones stéroïdiennes, les antifongiques, les rétinoïdes, les anti-inflammatoires stéroïdiens, les anti-inflammatoires non stéroïdiens, les anti-rétroviraux, les inhibiteurs de protéases, les antiacides, les inhibiteurs de la pompe à protons, les antiémétiques, les vitamines liposolubles, les médicaments du système cardiovasculaire, les antiagrégants plaquettaires, les anticancéreux, certains extraits végétaux et leurs principes actifs isolés ou dérivés, les immunosuppresseurs, les médicaments du système nerveux central, les antimigraineux, les antibiotiques et les antiparasitaires.

3. Composition selon la revendication 2, **caractérisée par le fait que** les hypolipidémiants sont choisis parmi le 1-méthyléthyl ester de l'acide 2-(4-(4-chlorobenzoyl)phénoxy)-2-méthyl-propanoïque (fénofibrate), le bézafibrate, le ciprofibrate, le gemfibrozil, le probucol, le tiadénol, la simvastatine, la mévastatine, la lovastatine, l'atorvastatine, la pravastatine, la fluvastatine, la cerivastatine et le mélinamide.

4. Composition selon la revendication 2, **caractérisée par le fait que** les hormones stéroïdiennes sont choisies parmi citer les oestrogènes dérivés et esters de l'estradiol, la progestérone, le danazol, la testostérone et les esters et dérivés de testostérone, les anti-androgènes, les inhibiteurs de la 5 α-réductase, les inhibiteurs compétitifs de la testostérone, les dérivés de la quinazoline et les agonistes/antagonistes non stéroïdiens des récepteurs oestrogéniques.

5. Composition selon la revendication 2, **caractérisée par le fait que** les antifongiques sont choisis parmi l'itraconazole, le miconazole, le kétoconazole, le fluconazole, la griséofulvine, l'amphotéricine B et la terbinafine.

6. Composition selon l'une quelconque des revendications précédentes, **caractérisée par le fait que** le principe actif lipophile est le fénofibrate, la progestérone ou l'itraconazole.

7. Composition selon l'une quelconque des revendications précédentes, **caractérisée par le fait que** le principe actif représente de 10 à 90 % en poids du poids total de la composition pharmaceutique.

8. Composition selon l'une quelconque des revendications précédentes, **caractérisée par le fait que** l'agent tensioactif est choisi parmi les composés présentant une valeur de la balance hydrophile-lipophile (HLB) supérieure ou égale à 15.

9. Composition selon la revendication 8, **caractérisée par le fait que** l'agent tensioactif est choisi parmi le laurylsulfate de sodium (HLB 40), les poloxamers (HLB 16-29), les macrogol éthers d'alcools organiques (HLB 15-18), et les sucrose-esters d'acides organiques (HLB 15-16).

10. Composition selon la revendication 9, **caractérisée par le fait que** l'agent tensioactif est le laurylsulfate de sodium.

11. Composition selon l'une quelconque des revendications précédentes, **caractérisée par le fait que** l'agent tensioactif représente de 1 à 10 % en poids du poids total de ladite composition.

12. Composition selon l'une quelconque des revendications précédentes, **caractérisée par le fait que** l'agent tensioactif se présente sous forme solide.

13. Composition selon la revendication 12, **caractérisée par le fait que** l'agent tensioactif est co-micronisé avec le principe actif.

14. Composition selon l'une quelconque des revendications précédentes, **caractérisée par le fait que** le rapport pondéral principe actif/agent tensioactif est compris entre 100/1 et 5/1.

15. Composition selon l'une quelconque des revendications précédentes, **caractérisée par le fait que** les polymères cationiques insolubles dans l'eau à pH supérieur ou égal à 5 sont choisis parmi les polymères acryliques contenant un groupe amine tertiaire.

16. Composition selon la revendication 15, **caractérisée par le fait que** lesdits polymères sont choisis parmi les polymères de type méthacrylate aminoalkyle solubles à pH inférieur à 5.

17. Composition selon la revendication 16, **caractérisée par le fait que** lesdits polymères sont choisis parmi le terpolymère de poly(diméthylaminoéthyl)méthacrylate, de méthyl méthacrylate et de butyl méthacrylate et le terpolymère de poly(diéthylaminoéthyl)méthacrylate, de méthyl méthacrylate et de butyl méthacrylate.

18. Composition selon l'une quelconque des revendications précédentes, **caractérisée par le fait que** le ou les polymères cationiques insolubles dans l'eau à pH supérieur ou égal à 5 représentent de 0,5 à 15 % en poids par rapport au poids total de la composition pharmaceutique.

19. Composition selon l'une quelconque des revendications précédentes, **caractérisée par le fait que** le rapport pondéral polymère cationique insoluble dans l'eau à pH supérieur ou égal à 5 / principe actif est compris entre 1/5 et 1/30.

20. Composition selon l'une quelconque des revendications précédentes, **caractérisée par le fait que** l'acide organique ou minéral est choisi parmi l'acide citrique, l'acide succinique, l'acide fumarique, l'acide acétique, l'acide phosphorique, l'acide sulfurique et l'acide chlorhydrique.

21. Composition selon l'une quelconque des revendications précédentes, **caractérisée par le fait que** l'acide organique ou minéral représente de 1 à 10 % en poids par rapport au poids total de la composition.

22. Composition selon l'une quelconque des revendications précédentes, **caractérisée par le fait que** le rapport pondéral acide organique ou minéral/polymère cationique insoluble dans l'eau à pH supérieur ou égal à 5 est compris entre 6/1 et 0,25/1.

23. Composition selon l'une quelconque des revendications précédentes, **caractérisée par le fait que** le ratio principe actif lipophile/acide minéral ou organique est compris entre 1/1 et 30/1.

24. Composition selon l'une quelconque des revendications précédentes, **caractérisée par le fait qu'**elle comprend, en poids par rapport au poids total de la composition :
- 40 à 80 % de fénofibrate à titre de principe actif lipophile,
- 2 à 10 % d'agent tensioactif,
- 2 à 10 % d'un terpolymère de poly(diméthylaminoéthyl)-méthacrylate, de méthyl méthacrylate et de butyl méthacrylate, et
- de 2,5 à 5 % d'un acide minéral ou organique.

25. Composition selon l'une quelconque des revendications précédentes, **caractérisée par le fait qu'**elle renferme du fénofibrate à titre de principe actif lipophile et qu'elle présente une dissolution d'au moins 50 % en 15 minutes, supérieure à 80 % en 30 minutes, supérieure à 85 % en 45 minutes et supérieure à 90 % en 60 minutes, telle que mesurée conformément à la méthode de la palette tournante à 75 tours/min selon la Pharmacopée Européenne, dans un milieu de dissolution constitué de laurylsulfate de sodium 0,1 M en solution aqueuse portée à 37°C.

26. Composition selon l'une quelconque des revendications précédentes, **caractérisée par le fait que** la phase interne renferme un ou plusieurs excipients choisis parmi les agents diluants et/ou liants, les agents désintégrants et les adjuvants de pulvérisation, de compression, de lubrification et d'écoulement des poudres.

27. Composition selon l'une quelconque des revendications précédentes, **caractérisée par le fait qu'**elle comprend en outre une phase externe comprenant un ou plusieurs excipients.

28. Procédé de préparation d'une composition pharmaceutique telle que définie à l'une quelconque des revendications 1 à 27, **caractérisé par le fait qu'**il comprend les étapes suivantes :
a) le mélange d'au moins un principe actif lipophile solide et micronisé, d'au moins un agent tensioactif, d'au moins un polymère cationique insoluble dans l'eau à pH supérieur ou égal à 5 et d'au moins un acide organique ou minéral,
b) la granulation ou l'atomisation du mélange obtenu ci-dessus à l'étape a),
c) l'addition éventuelle d'une phase externe comprenant un ou plusieurs excipients, puis
d) la compression ou la répartition en gélules du mélange obtenu à l'issue de l'étape b) ou c) lorsque cette dernière est réalisée.

29. Procédé selon la revendication 28, **caractérisé par le fait qu'**il comprend une étape préliminaire de co-micronisation du principe actif avec le ou les agents tensioactifs.

30. Procédé selon la revendication 28 ou 29, **caractérisé par le fait que** l'étape b) est réalisée par granulation et que le mélange des constituants lors de l'étape a) comprend les sous-étapes suivantes :
a1) la préparation d'une solution ou d'une suspension comprenant au moins acide organique ou minéral et au moins un polymère cationique insoluble dans l'eau à pH supérieur ou égal à 5, dans un liquide de granulation,
a2) la pulvérisation du mélange préparé ci-dessus à l'étape a1) sur le principe actif micronisé et préalablement mélangé à l'agent tensioactif solide ou co-micronisé avec ledit principe actif, à une température compatible avec la stabilité physique des substances engagées dans la formulation,
a3) la récupération des granulés fluidisés ainsi obtenus,
a4) le calibrage, des granulés fluidisés, et
a5) le séchage des granulés fluidisés.

31. Procédé selon la revendication 30, **caractérisé par le fait que** ledit polymère est un terpolymère de poly(diméthylaminoéthyl)méthacrylate, de méthyl méthacrylate et de butyl méthacrylate et que le liquide de granulation est un mélange propanol/acétone.

32. Procédé selon la revendication 28 ou 29, **caractérisé par le fait que** l'étape b) est réalisée par atomisation et que l'étape a) comprend les sous-étapes suivantes :
a'1) la préparation d'une solution acide ou tampon contenant un acide minéral ou organique et une base forte, ladite solution présentant un pH inférieur à 5,
a'2) la préparation d'une suspension, par addition à cette solution tampon, d'au moins un principe actif lipophile solide et micronisé, d'au moins un agent tensioactif éventuellement co-micronisé avec ledit principe actif et d'au moins un polymère cationique insoluble dans l'eau à pH supérieur ou égal à 5, sous agitation,
a'3) l'atomisation de ladite suspension,
a'4) la récupération du produit atomisé.

## Claims

1. A solid pharmaceutical composition intended to be administered orally, **characterized in that** it comprises, within one and the same phase (internal phase):
- at least one solid and micronized lipophilic active principle,
- at least one surfactant,
- at least one cationic polymer insoluble in water at pH greater than or equal to 5, and
- at least one organic or inorganic acid.

2. The composition as claimed in claim 1, **characterized in that** the lipophilic active principle is chosen from blood lipid-reducing agents, steroid hormones, antifungal agents, retinoids, steroidal anti-inflammatories, nonsteroidal anti-inflammatories, anti-retroviral agents, protease inhibitors, antacids, proton pump inhibitors, antiemetics, liposoluble vitamins, cardiovascular system drugs, anti-platelet aggregation agents, anticancer agents, certain plant extracts and their isolated or derived active principles, immunosuppressants, central nervous system drugs, antimigraine agents, antibiotics and antiparasitic agents.

3. The composition as claimed in claim 2, **characterized in that** the blood lipid-reducing agents are chosen from 2-(4-(4-chlorobenzoyl)phenoxy)-2-methylpropanoic acid 1-methylethyl ester (fenofibrate), bezafibrate, ciprofibrate, gemfibrozil, probucol, tiadenol, simvastatin, mevastatin, lovastatin, atorvastatin, pravastatin, fluvastatin, cerivastatin and melinamide.

4. The composition as claimed in claim 2, **characterized in that** the steroid hormones are chosen from derived estrogens and esters of estradiol, progesterone, danazol, testosterone and testosterone esters and derivatives, anti-androgens, 5α-reductase inhibitors, competitive inhibitors of testosterone, quinazoline derivatives and nonsteroidal agonists/ antagonists of estrogen receptors.

5. The composition as claimed in claim 2, **characterized in that** the antifungal agents are chosen from itraconazole, miconazole, ketoconazole, fluconazole, griseofulvin, amphotericin B and terbinafine.

6. The composition as claimed in any one of the preceding claims, **characterized in that** the lipophilic active principle is fenofibrate, progesterone or itraconazole.

7. The composition as claimed in any one of the preceding claims, **characterized in that** the active principle represents from 10 to 90% by weight of the total weight of the pharmaceutical composition.

8. The composition as claimed in any one of the preceding claims, **characterized in that** the surfactant is chosen from compounds having a hydrophilic-lipophilic balance (HLB) value greater than or equal to 15.

9. The composition as claimed in claim 8, **characterized in that** the surfactant is chosen from sodium lauryl sulfate (HLB 40), poloxamers (HLB 16-29), macrogol ethers of organic alcohols (HLB 15-18), and sucrose esters of organic acids (HLB 15-16).

10. The composition as claimed in claim 9, **characterized in that** the surfactant is sodium lauryl sulfate.

11. The composition as claimed in any one of the preceding claims, **characterized in that** the surfactant represents from 1 to 10% by weight of the total weight of said composition.

12. The composition as claimed in any one of the preceding claims, **characterized in that** the surfactant is in solid form.

13. The composition as claimed in claim 12, **characterized in that** the surfactant is comicronized with the active principle.

14. The composition as claimed in any one of the preceding claims, **characterized in that** the active principle/surfactant ratio by weight is between 100/1 and 5/1.

15. The composition as claimed in any one of the preceding claims, **characterized in that** the cationic polymers insoluble in water at pH greater than or equal to 5 are chosen from acrylic polymers comprising a tertiary amine group.

16. The composition as claimed in claim 15, **characterized in that** said polymers are chosen from polymers of aminoalkyl methacrylate type, soluble at pH below 5.

17. The composition as claimed in claim 16, **characterized in that** said polymers are chosen from the terpolymer of poly(dimethylaminoethyl)methacrylate, of methyl methacrylate and of butyl methacrylate and the terpolymer of poly(diethylaminoethyl)methacrylate, of methyl methacrylate and of butyl methacrylate.

18. The composition as claimed in any one of the preceding claims, **characterized in that** the cationic polymer(s) insoluble in water at pH greater than or equal to 5 represent(s) from 0.5 to 15% by weight relative to the total weight of the pharmaceutical composition.

19. The composition as claimed in any one of the preceding claims, **characterized in that** the cationic polymer insoluble in water at pH greater than or equal to 5/active principle ratio by weight is between 1/5 and 1/30.

20. The composition as claimed in any one of the preceding claims, **characterized in that** the organic or inorganic acid is chosen from citric acid, succinic acid, fumaric acid, acetic acid, phosphoric acid, sulfuric acid and hydrochloric acid.

21. The composition as claimed in any one of the preceding claims, **characterized in that** the organic or inorganic acid represents from 1 to 10% by weight relative to the total weight of the composition.

22. The composition as claimed in any one of the preceding claims, **characterized in that** the organic or inorganic acid/cationic polymer insoluble in water at pH greater than or equal to 5 ratio by weight is between 6/1 and 0.25/1.

23. The composition as claimed in any one of the preceding claims, **characterized in that** the lipophilic active principle/inorganic or organic acid ratio is between 1/1 and 30/1.

24. The composition as claimed in any one of the preceding claims, **characterized in that** it comprises, by weight relative to the total weight of the composition:
- 40 to 80% of fenofibrate as lipophilic active principle,
- 2 to 10% of surfactant,
- 2 to 10% of a terpolymer of poly(dimethylaminoethyl)-methacrylate, of methyl methacrylate and of butyl methacrylate, and
- from 2.5 to 5% of an inorganic or organic acid.

25. The composition as claimed in any one of the preceding claims, **characterized in that** it comprises fenofibrate as lipophilic active principle and **in that** it is at least 50% dissolved in 15 minutes, more than 80% dissolved in 30 minutes, more than 85% dissolved in 45 minutes and more than 90% dissolved in 60 minutes, as measured in accordance with the method using a paddle rotating at 75 rpm according to the European Pharmacopeia, in a dissolving medium consisting of 0.1M sodium lauryl sulfate in aqueous solution brought to 37°C.

26. The composition as claimed in any one of the preceding claims, **characterized in that** the internal phase comprises one or more excipients chosen from diluting agents and/or binders, disintegrating agents and adjuvants for spraying, tableting, lubrication and flow of powders.

27. The composition as claimed in any one of the preceding claims, **characterized in that** it also comprises an external phase comprising one or more excipients.

28. A method for preparing a pharmaceutical composition as defined in any one of claims 1 to 27, **characterized in that** it comprises the following steps:
a) mixing of at least one solid and micronized lipophilic active principle, of at least one surfactant, of at least one cationic polymer insoluble in water at pH greater than or equal to 5 and of at least one organic or inorganic acid,
b) granulation or atomization of the mixture obtained above in step a),
c) optional addition of an external phase comprising one or more excipients, then
d) tableting or dispensing into gelatin capsules of the mixture obtained at the end of step b) or c) when the latter is carried out.

29. The method as claimed in claim 28, **characterized in that** it comprises a preliminary step of comicronization of the active principle with the surfactant(s).

30. The method as claimed in claim 28 or 29, **characterized in that** step b) is carried out by granulation and **in that** the mixing of the constituents in step a) comprises the following substeps:
a1) preparing a solution or a suspension comprising at least one organic or inorganic acid and at least one cationic polymer insoluble in water at pH greater than or equal to 5, in a granulating liquid,
a2) spraying the mixture prepared above in step a1) onto the active principle which has been micronized and premixed with the solid surfactant or comicronized with said active principle, at a temperature compatible with the physical stability of the substances used in the formulation,
a3) recovering the fluidized granules thus obtained,
a4) calibrating the fluidized granules, and
a5) drying the fluidized granules.

31. The method as claimed in claim 30, **characterized in that** said polymer is a terpolymer of poly(dimethylaminoethyl)methacrylate, of methyl methacrylate and of butyl methacrylate and **in that** the granulating liquid is a propanol/acetone mixture.

32. The method as claimed in claim 28 or 29, **characterized in that** step b) is carried out by atomization and **in that** step a) comprises the following substeps:
a'1) preparing an acid or buffer solution comprising an inorganic or organic acid and a strong base, said solution having a pH of less than 5,
a'2) preparing a suspension by addition, to this buffer solution, of at least one solid and micronized lipophilic active principle, of at least one surfactant optionally comicronized with said active principle and of at least one cationic polymer insoluble in water at pH greater than or equal to 5, with stirring,
a'3) atomizing said suspension,
a'4) recovering the atomized product.

## Patentansprüche

1. Feste pharmazeutische Zusammensetzung, die für die Verabreichung auf oralem Weg bestimmt ist, **dadurch gekennzeichnet, dass** sie innerhalb ein und derselben Phase (internen Phase) enthält:
- mindestens einen festen und mikronisierten lipophilen Wirkstoff,
- mindestens ein Tensid,
- mindestens ein in Wasser unlösliches kationisches Polymer mit einem pH-Wert oberhalb oder gleich 5, und
- mindestens eine organische oder Mineralsäure.

2. Zusammensetzung nach Anspruch 1, **dadurch gekennzeichnet, dass** der lipophile Wirkstoff aus den hypolipämischen Mitteln, den Steroidhormonen, den Fungiziden, den Retinoiden, den Steroidentzündungshemmern, den Nicht-Steroid-Entzündungshemmern, den antiretroviralen Mitteln, den Proteaseinhibitoren, den säureneutralisierenden Mitteln, den Protonenpumpinhibitoren, den Mitteln gegen Erbrechen, den fettlöslichen Vitaminen, den Medikamenten des Herz-Gefäß-Systems, den Thrombozytenaggregationshemmern, den krebshemmenden Mitteln, bestimmten Pflanzenextrakten und ihren isolierten oder abgeleiteten Wirkstoffen, den Immunosuppressiva, den Medikamenten des zentralen Nervensystems, den Antimigränemitteln, den Antibiotika und den Antiparasitenmitteln ausgewählt ist.

3. Zusammensetzung nach Anspruch 2, **dadurch gekennzeichnet, dass** die hypolämischen Mittel aus dem 1-Methylethylester von 2-(4-(4-Chlorbenzoyl)phenoxy)-2-methyl-propionsäure (Fenofibrat), Bezafibrat, Ciprofibrat, Gemfibrozil, Probucol, Tiadenol, Simvastatin, Mevastatin, Lovastatin, Atorvastatin, Pravastatin, Fluvastatin, Cerivastatin und Melinamid ausgewählt sind.

4. Zusammensetzung nach Anspruch 2, **dadurch gekennzeichnet, dass** die Steroidhormone aus den abgeleiteten Östrogenen und Östradiolestern, Progesteron, Danazol, Testosteron und den Estern und Derivaten von Testosteron, den Antiandrogenen, den Inhibitoren der 5-α-Reduktase, den Konkurrenzinhibitoren von Testosteron, den Derivaten von Chinazolin und den nicht-steroiden Agonisten/Antagonisten der Östrogenrezeptoren ausgewählt sind.

5. Zusammensetzung nach Anspruch 2, **dadurch gekennzeichnet, dass** die Fungizide aus Itraconazol, Miconazol, Ketoconazol, Fluconazol, Griseofulvin, Amphotericin B und Terbinafin ausgewählt sind.

6. Zusammensetzung nach einem der vorangehenden Ansprüche, **dadurch gekennzeichnet, dass** der lipophile Wirkstoff Fenofibrat, Progesteron oder Itraconazol ist.

7. Zusammensetzung nach einem der vorangehenden Ansprüche, **dadurch gekennzeichnet, dass** der Wirkstoff 10 bis 90 Gewichts-% des Gesamtgewichts der pharmazeutischen Zusammensetzung darstellt.

8. Zusammensetzung nach einem der vorangehenden Ansprüche, **dadurch gekennzeichnet, dass** das Tensid aus den Verbindungen ausgewählt ist, die einen Wert des hydrophilen-lipophilen Gleichgewichts (HLB) aufweisen, der größer als oder gleich 15 ist.

9. Zusammensetzung nach Anspruch 8, **dadurch gekennzeichnet, dass** das Tensid aus Natriumlaurylsulfat (HLB 40), den Poloxameren (HLB 16-29), den organischen Macrogol-Alkoholethern (HLB 15-18) und den Saccharoseestern von organischen Säuren (HLB 15-16) ausgewählt ist.

10. Zusammensetzung nach Anspruch 9, **dadurch gekennzeichnet, dass** das Tensid Natriumlaurylsulfat ist.

11. Zusammensetzung nach einem der vorangehenden Ansprüche, **dadurch gekennzeichnet, dass** das Tensid 1 bis 10 Gewichts-% des Gesamtgewichts der Zusammensetzung darstellt.

12. Zusammensetzung nach einem der vorangehenden Ansprüche, **dadurch gekennzeichnet, dass** das Tensid in fester Form vorliegt.

13. Zusammensetzung nach Anspruch 12, **dadurch gekennzeichnet, dass** das Tensid mit dem Wirkstoff gemeinsam mikronisiert ist.

14. Zusammensetzung nach einem der vorangehenden Ansprüche, **dadurch gekennzeichnet, dass** das Gewichtsverhältnis Wirkstoff/Tensid zwischen 100/1 und 5/1 liegt.

15. Zusammensetzung nach einem der vorangehenden Ansprüche, **dadurch gekennzeichnet, dass** die in Wasser unlöslichen kationischen Polymere mit einem pH-Wert größer als oder gleich 5 aus den Acrylpolymeren ausgewählt sind, die eine tertiäre Amingruppe enthalten.

16. Zusammensetzung nach Anspruch 15, **dadurch gekennzeichnet, dass** die Polymere aus den löslichen Polymeren des Aminoalkylmethacrylat-Typs mit einem pH-Wert unterhalb 5 ausgewählt sind.

17. Zusammensetzung nach Anspruch 16, **dadurch gekennzeichnet, dass** die Polymere aus dem Poly(dimethylaminoethyl)methacrylat-, Methylmethacrylat- und Butylmethacrylat-Terpolymer und dem Poly(diethylaminoethyl)methacrylat-, Methylmethacrylat- und Butylmethacrylat-Terpolymer ausgewählt sind.

18. Zusammensetzung nach einem der vorangehenden Ansprüche, **dadurch gekennzeichnet, dass** das in Wasser unlösliche kationische Polymer oder die in Wasser unlöslichen kationischen Polymere mit einem pH-Wert größer als oder gleich 5 0,5 bis 15 Gewichts-% bezüglich des Gesamtgewichts der pharmazeutischen Zusammensetzung darstellt (darstellen).

19. Zusammensetzung nach einem der vorangehenden Ansprüche, **dadurch gekennzeichnet, dass** das Gewichtsverhältnis in Wasser unlösliches kationisches Polymer mit einem pH-Wert größer als oder gleich 5/Wirkstoff zwischen 1/5 und 1/30 liegt.

20. Zusammensetzung nach einem der vorangehenden Ansprüche, **dadurch gekennzeichnet, dass** die organische oder Mineralsäure aus Zitronensäure, Bernsteinsäure, Fumarsäure, Essigsäure, Phosphorsäure, Schwefelsäure und Salzsäure ausgewählt ist.

21. Zusammensetzung nach einem der vorangehenden Ansprüche, **dadurch gekennzeichnet, dass** die organische oder Mineralsäure 1 bis 10 Gewichts-% bezüglich des Gesamtgewichts der Zusammensetzung darstellt.

22. Zusammensetzung nach einem der vorangehenden Ansprüche, **dadurch gekennzeichnet, dass** das Gewichtsverhältnis organische oder Mineralsäure/in Wasser unlösliches kationisches Polymer mit einem pH-Wert größer als oder gleich 5 zwischen 6/1 und 0,25/1 liegt.

23. Zusammensetzung nach einem der vorangehenden Ansprüche, **dadurch gekennzeichnet, dass** das Verhältnis lipophiler Wirkstoff/Mineral- oder organische Säure zwischen 1/1 und 30/1 liegt.

24. Zusammensetzung nach einem der vorangehenden Ansprüche, **dadurch gekennzeichnet, dass** sie auf das Gewicht bezüglich des Gesamtgewichts der Zusammensetzung umfasst:
- 40 bis 80 % Fenofibrat als lipophilen Wirkstoff,
- 2 bis 10 % Tensid,
- 2 bis 10 % eines Poly(dimethylaminoethyl)-methacrylat-Methylmethacrylat- und Butylmethacrylat-Terpolymers, und
- 2,5 bis 5 % einer Mineral- oder organischen Säure.

25. Zusammensetzung nach einem der vorangehenden Ansprüche, **dadurch gekennzeichnet, dass** sie Fenofibrat als lipophilen Wirkstoff enthält und dass sie eine Auflösung von mindestens 50 % in 15 Minuten, höher als 80 % in 30 Minuten, höher als 85 % in 45 Minuten und höher als 90 % in 60 Minuten, wie gemäß dem Drehflügelverfahren mit 75 Umdrehungen/min gemäß dem Europäischen Arzneibuch gemessen, in einer Auflösungsumgebung, die aus Natriumlaurylsulfat 0,1 M in einer auf 37 °C gebrachten wässerigen Lösung besteht, aufweist.

26. Zusammensetzung nach einem der vorangehenden Ansprüche, **dadurch gekennzeichnet, dass** die interne Phase einen oder mehrere Exzipienten enthält, die aus den Verdünnungs- und/oder Bindemitteln, den Zerfallshilfsmitteln und den Pulverisierungs-, Verdichtungs-, Schmier- und Strömungshilfsmitteln für Pulver ausgewählt sind.

27. Zusammensetzung nach einem der vorangehenden Ansprüche, **dadurch gekennzeichnet, dass** sie außerdem eine externe Phase mit einem oder mehreren Exzipienten umfasst.

28. Verfahren zur Herstellung einer pharmazeutischen Zusammensetzung, wie in einem der Ansprüche 1 bis 27 definiert, **dadurch gekennzeichnet, dass** es die folgenden Schritte umfasst:
a) Mischen von mindestens einem festen und mikronisierten lipophilen Wirkstoff, von mindestens einem Tensid, von mindestens einem in Wasser unlöslichen kationischen Polymer mit einem pH-Wert größer als oder gleich 5 und von mindestens einer organischen oder Mineralsäure,
b) Granulieren oder Zerstäuben des vorstehend in Schritt a) erhaltenen Gemisches,
c) eventuelles Zugeben einer externen Phase mit einem oder mehreren Exzipienten, dann
d) Verdichten oder Verteilen des Gemisches in Gelatinekapseln, das am Ausgang von Schritt b) oder c), wenn dieser letztere ausgeführt wird, erhalten wird.

29. Verfahren nach Anspruch 28, **dadurch gekennzeichnet, dass** es einen Vorschritt der gemeinsamen Mikronisierung des Wirkstoffs mit dem Tensid oder den Tensiden umfasst.

30. Verfahren nach Anspruch 28 oder 29, **dadurch gekennzeichnet, dass** der Schritt b) durch Granulieren ausgeführt wird und dass das Mischen der Bestandteile in Schritt a) die folgenden Unterschritte umfasst:
a1) Herstellen einer Lösung oder einer Suspension mit mindestens einer organischen oder Mineralsäure und mindestens einem in Wasser unlöslichen kationischen Polymer mit einem pH-Wert größer als oder gleich 5 in einer Granulierungsflüssigkeit,
a2) Pulverisieren des vorstehend in Schritt a1) hergestellten Gemisches auf dem mikronisierten und vorher mit dem festen Tensid vermischten oder mit dem Wirkstoff gemeinsam mikronisierten Wirkstoff bei einer Temperatur, die mit der physikalischen Stabilität der in die Formulierung eingebundenen Substanzen kompatibel ist,
a3) Wiedergewinnen der so erhaltenen fluidisierten Granulate,
a4) Kalibrieren der fluidisierten Granulate, und
a5) Trocknen der fluidisierten Granulate.

31. Verfahren nach Anspruch 30, **dadurch gekennzeichnet, dass** das Polymer ein Poly(dimethylaminoethyl)methacrylat-, Methylmethacrylat- und Butylmethacrylat-Terpolymer ist und dass die Granulierungsflüssigkeit ein Propanol/Aceton-Gemisch ist.

32. Verfahren nach Anspruch 28 oder 29, **dadurch gekennzeichnet, dass** der Schritt b) durch Zerstäubung ausgeführt wird und dass der Schritt a) die folgenden Unterschritte umfasst:
a'1) Herstellen einer sauren oder Pufferlösung, die eine Mineral- oder organische Säure und eine starke Base enthält, wobei die Lösung einen pH-Wert aufweist, der geringer als 5 ist,
a'2) Herstellen einer Suspension durch Zugeben mindestens eines festen und mikronisierten lipophilen Wirkstoffs, mindestens eines Tensids, das eventuell mit dem Wirkstoff gemeinsam mikronisiert ist, und mindestens eines in Wasser unlöslichen kationischen Polymers mit einem pH-Wert größer als oder gleich 5 zu dieser Pufferlösung unter Rühren,
a'3) Zerstäuben der Suspension,
a'4) Wiedergewinnen des zerstäubten Produkts.
